(19) Europäisches Patentamt
European Patent Office
Office européen des brevets

(11) Veröffentlichungsnummer: **0 038 528**
**B1**

(12) **EUROPÄISCHE PATENTSCHRIFT**

(45) Veröffentlichungstag der Patentschrift: **08.08.84**

(51) Int. Cl.³: **C 07 D 471/04**, A 61 K 31/53

(21) Anmeldenummer: **81102906.5**

(22) Anmeldetag: **15.04.81**

(54) **Dihydro-as-triazino(5,6-c)chinolinderivate, Verfahren zu ihrer Herstellung und diese Verbindungen enthaltende Arzneimittel.**

(30) Priorität: **18.04.80 HU EG000939**

(43) Veröffentlichungstag der Anmeldung: **28.10.81 Patentblatt 81/43**

(45) Bekanntmachung des Hinweises auf die Patenterteilung: **08.08.84 Patentblatt 84/32**

(84) Benannte Vertragsstaaten: **AT BE CH DE FR GB IT LI NL SE**

(56) Entgegenhaltungen:
**DE - A - 2 216 241**
**DE - A - 2 322 394**
**DE - A - 2 322 418**
**DE - A - 2 322 486**

**Die Akte enthält technische Angaben, die nach dem Eingang der Anmeldung eingereicht wurden und die nicht in dieser Patentschrift enthalten sind.**

(73) Patentinhaber: **EGYT GYOGYSZERVEGYESZETI GYAR, Keresztúri ut 30-38, Budapest X (HU)**

(72) Erfinder: **Berenyi geb. Poldermann, Edit, Bartok Béla ut 36-38, Budapest XI. (HU)**
Erfinder: **Görög, Péter, Dr., Solymar u. 10, Budapest (HU)**
Erfinder: **Grasser, Katalin, Dr., Ferencz krt. 30, Budapest IX. (HU)**
Erfinder: **Kosoczky, Ibolya, Dr., Zolyomi ut 14, Budapest XI. (HU)**
Erfinder: **Kovács geb. Palotai, Agnes, Balkán u. 16, Budapest X. (HU)**
Erfinder: **Petöcz, Lujza, Dr., Rákoczi tér 2, Budapest VIII. (HU)**

(74) Vertreter: **Beszédes, Stephan G. Dr., Münchener Strasse 80a Postfach 1168, D-8060 Dachau (DE)**

**EP 0 038 528 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft neue Dihydro-as-triazino[4,5-c]chinolinderivate, ein Verfahren zu ihrer Herstellung und diese Verbindungen enthaltende Arzneimittel, insbesondere solche mit schmerzstillenden (analgetischen), entzündungshemmenden, krampfhemmenden (antikonvulsiven), beruhigenden (sedativen), narkosepotenzierenden und Tetrabenazin entgegenwirkenden Wirkungen.

1,2-Dihydro-as-triazino[5,6-c]chinoline, die in der 3-Stellung substituiert sind, sind bereits aus dem Fachschrifttum bekannt (deutsche Offenlegungsschriften 2 322 394 und 2 322 486). Dihydro-as-triazino[5,6-c]chinoline, welche in der 1-, 2- beziehungsweise 4-Stellung substituiert sind, wurden aber bisher nicht beschrieben.

Ferner sind aus der deutschen Offenlegungsschrift 2 216 241 in der 3-Stellung durch ein Chloratom, eine Aminogruppe, einen 2-Hydroxyäthylaminorest, einen Methoxyrest oder einen Diallylaminorest substituierte Triazino[5,6-c]chinoline bekannt. Als deren pharmakologische Wirkung ist die gegen Pilze und Hefen angegeben. Sie haben keine schmerzstillenden, entzündungshemmenden, krampfhemmenden, beruhigenden, narkosepotenzierenden und Tetrabenazin entgegenwirkende Wirkungen.

Weiterhin sind aus der deutschen Offenlegungschrift 2 322 418 3-Amino-as-triazino[6,5]-chinolin und sein 1-Oxyd (3-Amino-as-triazino[6,5-c]chinolin-1-oxyd) bekannt. Als pharmakologische Wirkungen dieser Verbindungen sind die entzündungshemmende und antimikrobielle Wirkung angegeben. Diese beiden Verbindungen haben keine schmerzstillenden, krampfhemmenden, beruhigenden, narkosepotenzierenden und Tetrabenazin entgegenwirkende Wirkungen.

Der Erfindung liegt die Aufgabe zugrunde, überlegene pharmakologische Wirkungen aufweisende neue Dihydro-as-triazino[5,6-c]chinolinderivate, ein Verfahren zur Herstellung derselben und diese Verbindungen enthaltende Arzneimittel zu schaffen.

Das Obige wurde überraschenderweise durch die Erfindung erreicht.

Gegenstand der Erfindung sind Dihydro-as-triazino[5,6-c]chinolinderivate der allgemeinen Formel

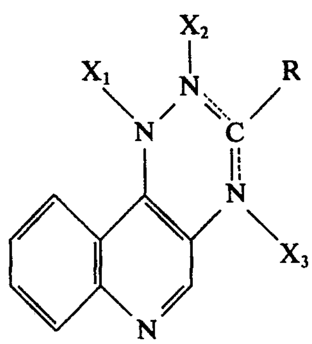

(I)

worin

R für einen Alkylrest mit 4, 5 oder 8 Kohlenstoffatomen oder einen Phenylrest steht und

$X_1$ ein Wasserstoffatom oder einen Acetylrest bedeutet und

entweder $X_2$ oder $X_3$ Wasserstoff oder einen Acetylrest darstellt und das andere von $X_2$ und $X_3$ keine Bedeutung hat und die gestrichelten Linien jeweils 1 chemische Bindung bedeuten, wobei nur entweder die eine oder die andere vorliegt,
mit der weiteren Maßgabe, daß falls $X_1$ für Wasserstoff steht, keines von $X_2$ und $X^3$ Wasserstoff bedeutet,

sowie ihre Säureadditionssalze.

Von sämtlichen Verbindungen der deutschen Offenlegungsschriften 2 216 241 und 2 322 418 unterscheiden sich die erfindungsgemäßen Verbindungen darin, daß sie Dihydroderivate (1,2- oder 1,4-Dihydroderivate) sind und keinen der Substituenten in der 3-Stellung der Verbindungen der genannten Druckschriften haben können.

Der Alkylrest mit 4, 5 oder 8 Kohlenstoffatomen, für welchen R stehen kann, kann geradkettig oder verzweigt, zum Beispiel ein n-Butyl-, sek.-Butyl-, tert.-Butyl-, Isobutyl-, n-Pentyl- oder n-Octylrest, sein.

Die Säureadditionssalze der erfindungsgemäßen Verbindungen sind zweckmäßig solche mit therapeutisch brauchbaren Säuren. Sie können solche mit anorganischen Säuren, insbesondere Salzsäure, Schwefelsäure oder Phosphorsäure, oder organischen Säuren, insbesondere Fumarsäure, Essigsäure, Maleinsäure, Citronensäure oder Milchsäure, sein.

Eine bevorzugte Gruppe der erfindungsgemäßen Dihydro-as-triazino[5,6-c]chinolinderivate sind diejenigen, bei welchen von den gestrichelten Linien die gestrichelte Linie zwischen dem Stickstoffatom in der 2-Stellung des Triazinringes und dem Kohlenstoffatom in der 3-Stellung desselben eine chemische Bindung darstellt, und $X_3$ einen Acetylrest bedeutet, das heißt 1,4-Dihydro-as-triazino[5,6-c]chinolinderivate der allgemeinen Formel

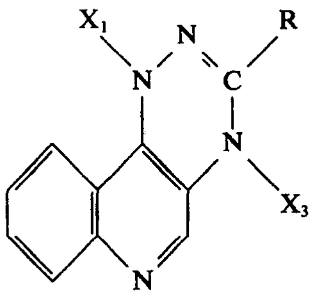

(Ia)

worin

$X_3$ einen Acetylrest bedeutet und
R und $X_1$ wie oben festgelegt sind,

sowie ihre Säureadditionssalze.

Eine weitere bevorzugte Gruppe der erfindungsgemäßen Dihydro-as-triazino[5,6-c]chinolinderivate sind diejenigen, bei welchen

$X_1$ für einen Acetylrest steht,
$X_2$ Wasserstoff oder einen Acetylrest bedeutet und

von den gestrichelten Linien die gestrichelte Linie zwischen dem Kohlenstoffatom in der 3-Stellung des Triazinringes und dem Stickstoffatom in der 4-Stellung desselben eine chemische Bindung darstellt,

das heißt 1,2-Dihydro-as-triazino[5,6-c]chinolinderivate der allgemeinen Formel

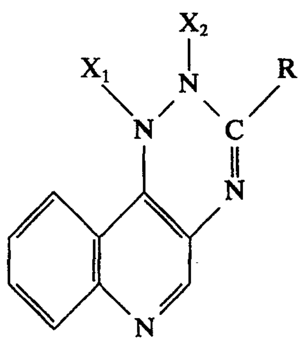

(Ib)

worin

$X_1$ für einen Acetylrest steht,
$X_2$ Wasserstoff oder einen Acetylrest bedeutet und
R wie oben festgelegt ist,

sowie ihre Säureadditionssalze.

Besonders bevorzugte erfindungsgemäße Verbindungen sind
1-Acetyl-3-n-pentyl-1,2-dihydro-as-triazino[5,6-c]chinolin,
4-Acetyl-3-phenyl-1,4-dihydro-as-triazino[5,6-c]chinolin, 1-Acetyl-3-n-butyl-1,
2-dihydro-as-triazino[5,6-c]chinolin, 1-Acetyl-3-n-octyl-1,2-dihydro-as-triazino[5,6-c]chinolin,
1-Acetyl-3-isobutyl-1,2-dihydro-as-triazino[5,6-c]chinolin,
1-Acetyl-3-(3'-methylbut-1'-yl)-1,2-dihydro-as-triazino[5,6-c]chinolin und
1-Acetyl-3-tert.-butyl-1,2-dihydro-as-triazino[5,6-c]chinolin.

Gegenstand der Erfindung ist auch ein Verfahren zur Herstellung der erfindungsgemäßen Verbindungen, welches dadurch gekennzeichnet ist, daß in an sich bekannter Weise Säureadditionssalze

von Dihydro-as-triazino[5,6-c]chinolinderivaten der allgemeinen Formel

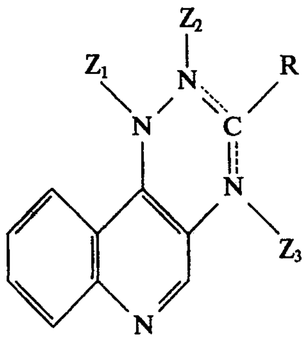

(II)

worin

R wie oben festgelegt ist und
$Z_1$ für Wasserstoff steht und

entweder $Z_2$ oder $Z_3$ Wasserstoff bedeutet und
das andere von $Z_2$ und $Z_3$ keine Bedeutung hat und
die gestrichelten Linien jeweils 1 chemische Bindung bedeuten, wobei nur entweder die eine oder die andere vorliegt,

mit Essigsäure und/oder acetylierungsaktiven Derivaten derselben als Acetylierungsmitteln acetyliert werden und gegebenenfalls erhaltene Monoacetyldihydro-as-triazino[5,6-c]chinolinderivate der allgemeinen Formel I beziehungsweise Säureadditionssalze derselben, gegebenenfalls nach Freisetzen der ersteren aus den letzteren beziehungsweise Überführen der letzteren in die ersteren, durch weiteres Acetylieren mit Essigsäure und/oder acetylierungsaktiven Derivaten derselben als Acetylierungsmitteln in Diacetyldihydro-as-triazino[5,6-c]chinolinderivate der allgemeinen Formel I beziehungsweise Säureadditionssalze derselben überführt werden, worauf in an sich bekannter Weise gegebenenfalls die erhaltenen Dihydro-as-triazino[5,6-c]chinolinderivate der allgemeinen Formel I mit anorganischen oder organischen Säuren in Säureadditionssalze überführt werden beziehungsweise gegebenenfalls die erhaltenen Säureadditionssalze der Dihydro-as-triazino[5,6-c]chinolinderivate der allgemeinen Formel I mit Basen in die freien Dihydro-as-triazino[5,6-c]chinolinderivate der allgemeinen Formel I oder in andere Säureadditionssalze überführt werden.

Vorzugsweise werden zum Acetylieren acetylierungsaktive Derivate der Essigsäure als Acetylierungsmittel verwendet.

Besonders bevorzugt werden als acetylierungsaktive Derivate der Essigsäure Carbonsäureanhydride der allgemeinen Formel

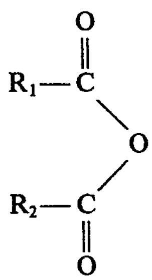

(III)

worin $R_1$ und $R_2$ unabhängig voneinander Wasserstoff beziehungsweise Methylreste bedeuten, verwendet.

Vorzugsweise werden die Acetylierungsreaktionen des erfindungsgemäßen Verfahrens bei Verwendung von acetylierungsaktiven Derivaten der Essigsäure in Gegenwart von Essigsäure durchgeführt.

Auch ein Überschuß des Acetylierungsmittels kann als Lösungsmittel beziehungsweise Verdünnungsmittel verwendet werden. Die Acetylierung kann aber auch in inerten Lösungsmitteln, welche mit den Reaktionsteilnehmern nicht reagieren, durchgeführt werden.

Als Reaktionstemperatur werden im allgemeinen 0 bis 200°C, vorzugsweise 40 bis 150°C, angewandt. Am meisten bevorzugt wird die Acetylierung der als Ausgangsverbindungen verwendeten Dihydro-as-triazino[5,6-c]chinolinderivate der allgemeinen Formel II beim Siedepunkt des Reaktionsgemisches durchgeführt.

Die Ausgangsverbindungen Dihydro-as-triazino[5,6-c)chinolinderivate der allgemeinen Formel II werden zweckmäßig mit 2- bis 3fachen äquimolaren Mengen der Acetylierungsmittel umgesetzt.

Das aus dem abgekühlten Reaktionsgemisch ausgeschiedene Produkt besteht im allgemeinen aus dem jeweiligen Säureadditionssalz des jeweiligen Monoacetyl-1,2-dihydro-as-triazino[5,6-c]chinolinderivates der allgemeinen Formel I. Die Monoacetyl-1,2-dihydro-as-triazino[5,6-c]chinolinderivate der allgemeinen Formel I beziehungsweise ihre Säureadditionssalze können durch eine weitere Acetylierungsstufe in Diacetyl-1,2-dihydro-as-triazino[5,6-c]chinolinderivate der allgemeinen Formel I beziehungsweise ihre Säureadditionssalze überführt werden. Zweckmäßig wird diese zweite Acetylierungsstufe mit einem größeren Acetylierungsmittelüberschuß in der oben angegebenen Weise durchgeführt. In diesem Fall können vor der Acetylierung die Monoacetyl-1,2-dihydro-as-triazino[5,6-c]chinolinderivate der allgemeinen Formel I zunächst aus ihren Säureadditionssalzen mit Basen, zum Beispiel Natriumbicarbonat, freigesetzt werden.

In manchen Fällen werden Monoacetyl-1,2-dihydro-as-triazino[5,6-c]chinolinderivate und Diacetyl-1,2-dihydro-as-triazino[5,6-c]chinolinderivate der allgemeinen Formel I nebeneinander je nach den Reaktionsbedingungen, wie dem Überschuß des Acetylierungsmittels und der Reaktionszeit, erhalten. Die Bildung der Monoacetyl-1,2-dihydro-as-triazino[5,6-c]chinolinderivate wird durch kürzere Reaktionszeiten und kleinere Überschüsse des Acetylierungsmittels begünstigt. Dagegen kann die Bildung der Diacetyl-1,2-dihydro-as-triazino[5,6-c]chinolinderivate (Diacetylierung) durch die Anwendung von längeren Reaktionszeiten beziehungsweise höheren Überschüssen des Acetylierungsmittels gefördert werden.

Die als Ausgangsverbindungen verwendeten Dihydro-as-triazino[5,6-c]chinolinderivate der allgemeinen Formel II können auf die in den deutschen Offenlegungsschriften 2 322 394 und 2 322 486 beschriebene Weise hergestellt worden sein.

Ferner sind erfindungsgemäß Arzneimittel, welche 1 oder mehr der erfindungsgemäßen Verbindungen als Wirkstoff beziehungsweise Wirkstoffe, gegebenenfalls zusammen mit 1 oder mehr inerten festen oder flüssigen Träger(n), enthalten, vorgesehen.

Die erfindungsgemäßen Verbindungen haben nämlich wie bereits erwähnt wertvolle pharmakologische Wirkungen, insbesondere schmerzstillende, entzündungshemmende, krampfhemmende, beruhigende, narkosepotenzierende und Tetrabenazin entgegenwirkende Wirkungen.

Zunächst wurde die akute Toxizität von erfindungsgemäßen Verbindungen und der Vergleichssubstanzen 2-Methyl-2-n-propylpropan-1,3-dioldicarbamat [Meprobamat], 4-n-Butyl-1,2-diphenylpyrazolidin-3,5-dion [Phenylbutazon], p-Hydroxyacetanilid [Paracetamol], 3,5,5-Trimethyloxazolidin-2,4-dion [Trimethadion] und 5-(3'-Dimethylaminopropyliden)-dibenz-[a,d]-1,4-cycloheptadien [Amitryptilin] an Mäusen beiderlei Geschlechtes mit Gewichten von 18 bis 22 g bei peroraler Verabreichung ermittelt. Die erhaltenen $LD_{50}$-Werte sind in der folgenden Tabelle I zusammengestellt.

Tabelle I

| Verbindung Bezeichnung | Beispiel | Peroraler $LD_{50}$-Wert in mg/kg |
|---|---|---|
| 1-Acetyl-3-n-pentyl-1,2-dihydro-as-triazino-[5,6-c]chinolin | 1 | 2000 |
| 4-Acetyl-3-phenyl-1,4-dihydro-as-triazino-[5,6-c]chinolin | 3 | 2000 |
| 1-Acetyl-3-n-butyl-1,2-dihydro-as-triazino-[5,6-c]chinolin | 6 | 2000 |
| 1-Acetyl-3-n-octyl-1,2-dihydro-as-triazino-[5,6-c]chinolin | 7 | 2000 |
| 1-Acetyl-3-isobutyl-1,2-dihydro-as-triazino-[5,6-c]chinolin | 8 | 2000 |
| 1-Acetyl-3-tert.-butyl-1,2-dihydro-as-triazino-[5,6-c]chinolin | 9 | 2000 |
| 1-Acetyl-3-(3'-methylbut-1'-yl)-1,2-dihydro-as-triazino[5,6-c]chinolin | 10 | 2000 |
| 2-Methyl-2-n-propylpropan-1,3-dioldicarbamat [Meprobamat] | Vergleichssubstanz A | 1100 |

Tabelle I (Fortsetzung)

| Verbindung Bezeichnung | Beispiel | Peroraler $LD_{50}$-Wert in mg/kg |
|---|---|---|
| 4-n-Butyl-1,2-diphenylpyrazolidin-3,5-dion [Phenylbutazon] | Vergleichssubstanz B | 1000 |
| p-Hydroxyacetanilid [Paracetamol] | Vergleichssubstanz C | 540 |
| 3,5,5-Trimethyloxazolidin-2,4-dion [Trimethadion] | Vergleichssubstanz D | 2050 |
| 5-(3'-Dimethylaminopropyliden)-dibenz-[a,d]-1,4-cycloheptadien [Amitryptilin] | Vergleichssubstanz E | 225 |

Die schmerzstillende beziehungsweise analgetische Wirkung von erfindungsgemäßen Verbindungen und der anerkannt gut wirksamen Vergleichssubstanzen p-Hydroxyacetanilid [Paracetamol] und 4-n-Butyl-1,2-diphenylpyrazolidin-3,5-dion [Phenylbutazon] gleicher Wirkungsrichtung wurde mit Hilfe des Essigsäure-»Schmerzkrümmungs«-Versuches (Essigsäure-»Writhing«-Versuches) an Mäusen untersucht. Die Schmerzkrümmungs-Reaktionen wurden 5 bis 10 Minuten nach intraperitonealer Verabreichung von 0,4 $cm^3$ einer 0,5%igen Essigsäurelösung gezählt. Nach der Behandlung wurde die innerhalb 5 Minuten festgestellte Zahl der Schmerzkrümmungen als Prozente der entsprechenden Blindversuchs- beziehungsweise Kontrollwerte ausgedrückt. Die Tiere wurden jeweils 1 Stunde vor der Verabreichung der Essigsäure mit der zu untersuchenden Verbindung beziehungsweise dem keinen Wirkstoff enthaltenden Träger peroral behandelt. Die erhaltenen Ergebnisse sind in der folgenden Tabelle II, in welcher sowohl die $ED_{50}$-Werte als auch die therapeutischen Indices angegeben sind, zusammengestellt.

Tabelle II

| Verbindung Bezeichnung | Beispiel | Peroraler $ED_{50}$-Wert in mg/kg | Therapeutischer Index $\frac{LD_{50}\text{-Wert}}{ED_{50}\text{-Wert}}$ |
|---|---|---|---|
| 1-Acetyl-3-n-pentyl-1,2-dihydro-as-triazino[5,6-c]chinolin | 1 | 125 | 16 |
| 1-Acetyl-3-(3'-methylbut-1'-yl)-1,2-dihydro-as-triazino[5,6-c]chinolin | 10 | 100 | 20 |
| p-Hydroxyacetanilid [Paracetamol] | Vergleichssubstanz C | 180 | 3 |
| 4-n-Butyl-1,2-diphenylpyrazolidin-3,5-dion [Phenylbutazon] | Vergleichssubstanz B | 65 | 15,4 |

Die entzündungshemmende Wirkung von erfindungsgemäßen Verbindungen und der anerkannt gut wirksamen Vergleichssubstanz 4-n-Butyl-1,2-diphenylpyrazolidin-3,5-dion [Phenylbutazon] gleicher Wirkungsrichtung wurde auf Grund der Hemmung von in Füßen von Ratten künstlich hervorgerufenen Ödemen bestimmt. Es wurde 0,1 $cm^3$ einer 1%igen Carraghenin-Suspension jeweils in die Fußsohle eines der Hinterglieder der Tiere injiziert. Die zu untersuchende Verbindung wurde den Tieren jeweils 1 Stunde vor der Verabreichung der Carraghenin-Suspension peroral verabreicht. Den Tieren der Blindversuchs- beziehungsweise Kontrollgruppe wurde der keinen Wirkstoff enthaltende Träger peroral verabreicht. Das Volumen der Sohle wurde vor der Verabreichung der Entzündungen hervorrufenden Suspension und 3 Stunden danach mit Hilfe eines Quecksilberplethysmometers gemessen. In der folgenden Tabelle III sind die $ED_{50}$-Werte und die therapeutischen Indices zusammengestellt.

Tabelle III

| Verbindung Bezeichnung | Beispiel | Peroraler $ED_{50}$-Wert in mg/kg | Therapeutischer Index $\frac{LD_{50}\text{-Wert}}{ED_{50}\text{-Wert}}$ |
|---|---|---|---|
| 1-Acetyl-3-n-pentyl-1,2-dihydro-as-triazino[5,6-c]chinolin | 1 | 100 | 20 |
| 1-Acetyl-3-n-butyl-1,2-dihydro-as-triazino[5,6-c]chinolin | 6 | 36 | 56 |
| 1-Acetyl-3-(3'-methylbut-1'-yl)-1,2-dihydro-as-triazino[5,6-c]chinolin | 10 | 200 | 10 |
| 4-n-Butyl-1,2-diphenylpyrazolidin-3,5-dion [Phenylbutazon] | Vergleichssubstanz B | 100 | 10 |

Im Gegensatz zu den Verbindungen der deutschen Offenlegungsschrift 2 216 241, welche überhaupt keine entzündungshemmende Wirkung haben, haben also die erfindungsgemäßen Verbindungen eine bedeutende entzündungshemmende Wirkung. Gegenüber dem 3-Amino-as-triazino[6,5]chinolin der deutschen Offenlegungsschrift 2 322 418, welches hinsichtlich der entzündungshemmenden Wirkung einen therapeutischen Index von nur 3,67 hat, und gegenüber dem 3-Amino-as-triazino[6,5]chinolin-1-oxyd dieser Druckschrift, dessen therapeutischer Index hinsichtlich der entzündungshemmenden Wirkung noch schlechter ist, erscheinen somit die erfindungsgemäßen Verbindungen überlegen.

Die krampfhemmende Wirkung von erfindungsgemäßen Verbindungen und der anerkannt gut wirksamen Vergleichssubstanz 3,5,5-Trimethyloxazolidin-2,4-dion [Trimethadion] gleicher Wirkungsrichtung wurde auf Grund der Hemmung des durch Elektroschock hervorgerufenen Krampfes an Mäusen mit Gewichten von 20 bis 25 g untersucht. Der Elektroschock wurde durch Anwendung von an der Hornhaut angebrachten (kornealen) Elektroden 0,4 Sekunden lang ausgelöst. Die Stromintensität war 45 mA und die Frequenz des Stromes betrug 50 Hz.

Als Kriterium der krampfhemmenden Wirkung wurde die vollkommene Verhinderung der tonischen Streckung (Extension) der Hinterglieder bewertet.

Die zu untersuchenden Verbindungen wurden den Tieren 1 Stunde vor dem Elektroschock peroral verabreicht. Die erhaltenen Ergebnisse sind in der folgenden Tabelle IV, in welcher sowohl die $ED_{50}$-Werte als auch die therapeutischen Indices angegeben sind, zusammengestellt.

Tabelle IV

| Verbindung Bezeichnung | Beispiel | Peroraler $ED_{50}$-Wert in mg/kg | Therapeutischer Index $\frac{LD_{50}\text{-Wert}}{ED_{50}\text{-Wert}}$ |
|---|---|---|---|
| 1-Acetyl-3-n-butyl-1,2-dihydro-as-triazino[5,6-c]chinolin | 6 | 160 | 12,5 |
| 1-Acetyl-3-isobutyl-1,2-dihydro-as-triazino[5,6-c]chinolin | 8 | 200 | 10 |
| 3,5,5-Trimethyloxyzolidin-2,4-dion [Trimethadion] | Vergleichssubstanz D | 400 | 5,1 |

Ferner wurde die krampfhemmende Wirkung von erfindungsgemäßen Verbindungen und der anerkannt gut wirksamen Vergleichssubstanz 3,5,5-Trimethyloxazolidin-2, 4-dion [Trimethadion] gleicher Wirkungsrichtung auf Grund der Verhinderung der durch 6,7,8,9-Tetrahydro-5H-tetrazoloazepin [Pentamethylentetrazol] hervorgerufenen Krämpfe an Gruppen von je 6 Mäusen untersucht. Das 6,7,8,9-Tetrahydro-5H-tetrazoloazepin wurde den Tieren in Dosen von 125 mg/kg intraperitoneal verabreicht und die tonische Streckung (Extension) der Hinterglieder wurde registriert. Die zu untersuchenden Verbindungen wurden den Mäusen 1 Stunde vor der Verabreichung des 6,7,8,9-Tetrahydro-5H-tetrazoloazepines peroral verabreicht.

Die erhaltenen Ergebnisse sind in der folgenden Tabelle V, in welcher sowohl die $ED_{50}$-Werte als auch die therapeutischen Indices angegeben sind, zusammengestellt.

Tabelle V

| Verbindung Bezeichnung | Beispiel | Peroraler $ED_{50}$-Wert in mg/kg | Therapeutischer Index $\frac{LD_{50}\text{-Wert}}{ED_{50}\text{-Wert}}$ |
|---|---|---|---|
| 1-Acetyl-3-n-butyl-1,2-dihydro-as-triazino[5,6-c]chinolin | 6 | 90 | 22,2 |
| 1-Acetyl-3-n-octyl-1,2-dihydro-as-triazino[5,6-c]chinolin | 7 | 350 | 5,7 |
| 1-Acetyl-3-n-isobutyl-1,2-dihydro-as-triazino[5,6-c]chinolin | 8 | 72 | 27,8 |
| 1-Acetyl-3-tert.-butyl-1,2-dihydro-as-triazino[5,6-c]chinolin | 9 | 165 | 12,1 |
| 3,5,5-Trimethyloxazolidin-2,4-dion [Trimethadion] | Vergleichssubstanz D | 490 | 4,2 |

Die mit 5-(1'-Cyclohexenyl)-1,5-dimethylbarbitursäure [Hexobarbital] hervorgerufene narkosepotenzierende Wirkung von erfindungsgemäßen Verbindungen und der anerkannt gut wirksamen Vergleichssubstanz 2-Methyl-2-n-propylpropan-1,3-dioldicarbamat [Meprobamat] gleicher Wirkungsrichtung wurde an Gruppen von je 6 Mäusen untersucht. Die zu untersuchenden Verbindungen wurden den Mäusen peroral verabreicht. Nach 1 Stunde wurde den Mäusen je eine Dosis von 40 mg/kg 5-(1'-Cyclohexenyl)-1,5-dimethylbarbitursäure [Hexobarbital] intravenös verabreicht. Es wurden diejenigen Tiere als positiv reagierend bewertet, deren Schlafdauer den durchschnittlichen Wert der Blindversuchs- beziehungsweise Kontrolltiere um 150% übertraf. Die Zahl der Tiere mit positiver Reaktion wurde mit der Zahl sämtlicher behandelten Tiere verglichen. Die berechneten $ED_{50}$-Werte und die therapeutischen Indices sind in der folgenden Tabelle VI zusammengestellt.

Tabelle VI

| Verbindung Bezeichnung | Beispiel | Peroraler $ED_{50}$-Wert in mg/kg | Therapeutischer Index $\frac{LD_{50}\text{-Wert}}{ED_{50}\text{-Wert}}$ |
|---|---|---|---|
| 1-Acetyl-3-n-pentyl-1,2-dihydro-as-triazino[5,6-c]chinolin | 1 | 200 | 10 |
| 4-Acetyl-3-phenyl-1,4-dihydro-as-triazino[5,6-c]chinolin | 3 | 150 | 13,3 |
| 1-Acetyl-3-n-butyl-1,2-dihydro-as-triazino[5,6-c]chinolin | 6 | 125 | 16 |
| 1-Acetyl-3-isobutyl-1,2-dihydro-as-triazino[5,6-c]chinolin | 8 | 70 | 28,6 |
| 1-Acetyl-3-(3'-methylbut-1'-yl)-1,2-dihydro-as-triazino[5,6-c]chinolin | 10 | 100 | 20 |
| 2-Methyl-2-n-propylpropan-1,3-dioldicarbamat [Meprobamat] | Vergleichssubstanz A | 260 | 4,2 |

Die Tetrabenazin entgegenwirkende Wirkung von erfindungsgemäßen Verbindungen und der anerkannt gut wirksamen Vergleichssubstanz 5-(3'-Dimethylaminopropyliden)-dibenz-[a,d]-1,4-cycloheptadien [Amitryptilin] gleicher Wirkungsrichtung wurde an Gruppen von je 10 Mäusen untersucht. Die zu untersuchenden Verbindungen wurden den Tieren peroral verabreicht. Nach 30 Minuten wurden den Mäusen je 50 mg/kg 3-Isobutyl-9,10-dimethoxy-1,2,3,4,6,7-hexahydrobenzo[a]chinolizin-2-on {Tetrabenazin} intraperitoneal verabreicht. Die Zahl der Tiere mit geschlossenen Augenlidern wurde nach 30, 60, 90 und 120 Minuten festgestellt. Die Angaben der einzelnen Meßzeitpunkte wurden addiert und die auf den Blind- beziehungsweise Kontrollversuch bezogene Hemmung wurde berechnet. Die erhaltenen $ED_{50}$-Werte und die therapeutischen Indices sind in der folgenden Tabelle VII zusammengestellt.

Tabelle VII

| Verbindung Bezeichnung | Beispiel | Peroaler $ED_{50}$-Wert in mg/kg | Therapeutischer Index $\frac{LD_{50}\text{-Wert}}{ED_{50}\text{-Wert}}$ |
|---|---|---|---|
| 1-Acetyl-3-isobutyl-1,2-dihydro-as-triazino[5,6-c]chinolin | 8 | 20 | 100 |
| 1-Acetyl-3-(3'-methylbut-1'-yl)-1,2-dihydro-as-triazino[5,6-c]chinolin | 10 | 14,5 | 138 |
| 1-Acetyl-3-(1'-äthylprop-1'-yl)-1,2-dihydro-as-triazino[5,6-c]chinolin | 11 | 54 | 37 |
| 5-(3'-Dimethylaminopropyliden)-dibenz-[a,d]-1,4-cycloheptadien [Amitryptilin] | Vergleichssubstanz E | 12 | 19 |

Aus den obigen Tabellen I bis VII geht eindeutig die therapeutische Überlegenheit der erfindungsgemäßen Verbindungen gegenüber den Vergleichssubstanzen hervor.

Die erfindungsgemäßen Verbindungen können in Form von Arzneimittelpräparaten vorliegen. Diese können nach an sich bekannten Verfahren der pharmazeutischen Industrie bereitet worden sein.

Die erfindungsgemäßen Arzneimittelpräparate können vorteilhaft in Form von peroral zu verabreichenden Zubereitungsformen, zum Beispiel Tabletten, Kapseln, Dragees, Lösungen oder Suspensionen, oder parenteral zu verabreichenden Zubereitungsformen, zum Beispiel sterilen Lösungen oder Suspensionen, vorliegen.

Die peroral zu verabreichenden Arzneimittelpräparate können als Träger beispielsweise verschiedene übliche Bindemittel, zum Beispiel Gelatine, Sorbit und/oder Polyvinylpyrrolidon, Füllstoffe, zum Beispiel Milchzucker, Rohrzucker, Stärke und/oder Calciumphosphat, Hilfsstoffe, zum Beispiel Magnesiumstearat, Talk, Polyäthylenglykole und/oder Siliciumdioxyd, und/oder Vernetzungsmittel, zum Beispiel Natriumlaurylsulfat, enthalten.

Die flüssigen Arzneimittelpräparate können als Träger verschiedene Suspendiermittel, zum Beispiel Sorbit, Rohrzuckerlösung, Gelatine und/oder Carboxymethylcellulose, Emulgiermittel, zum Beispiel Öle, ölige Ester, Glycerin, Propylenglykol und/oder Äthanol, und/oder Konservierungsmittel, zum Beispiel p-Hydroxybenzoesäuremethylester und/oder p-Hydroxybenzoesäure-n-propylester, enthalten.

Die erfindungsgemäßen Arzneimittelpräparate können auch an sich bekannte Geschmackskorrigentien und Farbstoffe enthalten.

Die Erfindung wird an Hand der folgenden Beispiele näher erläutert.

## Beispiel 1

### 1-Acetyl-3-n-pentyl-1,2-dihydro-as-triazino[5,6-c]chinolin

Es wurden 13,0 g (0,045 Mol) 3-n-Pentyl-1,2-dihydro-as-triazino[5,6-c]chinolinhydrochlorid 15 Minuten lang mit 30 $cm^3$ Essigsäureanhydrid zum Sieden erhitzt. Die Ausgangsverbindung 3-n-Pentyl-1,2-dihydro-as-triazino[5,6-c]chinolinhydrochlorid löste sich nach 5 Minuten langem Sieden, und nach weiteren 5 Minuten begann die Ausscheidung des Produktes in Form von roten Kristallen. Die ausgeschiedenen Kristalle wurden nach dem Abkühlen der Suspension abfiltriert und mit Äthylacetat gewaschen.

So wurden 11,2 g (75,3% der Theorie, bezogen auf das eingesetzte 3-n-Pentyl-1,2-dihydro-as-triazino[5,6-c]chinolinhydrochlorid) 1-Acetyl-3-n-pentyl-1,2-dihydro-as-triazino[5,6-c]chinolinhydrochlorid mit einem Schmelzpunkt von 212 bis 213° C erhalten.

6,0 g (0,018 Mol) dieses 1-Acetyl-3-n-pentyl-1,2-dihydro-as-triazino[5,6-c]chinolinhydrochlorides wurden in 40 $cm^3$ Wasser suspendiert, und der pH-Wert der Suspension wurde mit konzentrierter Natriumbicarbonatlösung auf 7 eingestellt. Die erhaltenen gelben Kristalle wurden abfiltriert und mit Wasser gewaschen.

So wurden 5,26 g (98,5% der Theorie, bezogen auf das eingesetzte 1-Acetyl-3-n-pentyl-1,2-dihydro-as-triazino[5,6-c]chinolinhydrochlorid, beziehungsweise 74,2% der Theorie, bezogen auf das eingesetzte 3-n-Pentyl-1,2-dihydro-as-triazino[5, 6-c]chinolinhydrochlorid), 1-Acetyl-3-n-pentyl-1,2-dihydro-as-triazino[5,6-c]chinolin mit einem Schmelzpunkt von 178 bis 180° C erhalten.

## Beispiel 2

### 1,2-Diacetyl-3-n-pentyl-1,2-dihydro-as-triazino[5,6-c]chinolin

Es wurden 9,5 g (0,032 Mol) wie im Beispiel 1 beschrieben erhaltenes 1-Acetyl-3-n-pentyl-1,2-dihydro-as-triazino[5,6-c]chinolin 4 Stunden lang mit 85 $cm^3$ Essigsäureanhydrid zum Sieden erhitzt, und die erhaltene Lösung wurde in Wasser eingegossen. Die ausgeschiedenen hellgelben Kristalle wurden abfiltriert und mit Wasser gewaschen.

So wurden 9,25 g (85,3% der Theorie, bezogen auf das eingesetzte 1-Acetyl-3-n-pentyl-1,2-dihydro-as-triazino[5,6-c]chinolin) 1,2-Diacetyl-3-n-pentyl-1,2-dihydro-as-triazino[5,6-c]chinolin mit einem Schmelzpunkt von 116 bis 117° C erhalten.

## Beispiel 3

### 4-Acetyl-3-phenyl-1,4-dihydro-as-triazino[5,6-c]chinolin

Es wurden 10,0 g (0,034 Mol) 3-Phenyl-1,4-dihydro-as-triazino[5,6-c]chinolinhydrochlorid 1 Stunde lang mit 20 $cm^3$ Essigsäureanhydrid zum Sieden erhitzt. Die dunkelviolette Farbe der Ausgangsverbindung ist während der Reaktion verschwunden, und es schieden sich ziegelfarbige Kristalle aus.

So wurden 6,2 g (54,3% der Theorie, bezogen auf das eingesetzte 3-Phenyl-1,4-dihydro-as-triazino[5,6-c]chinolinhydrochlorid), 4-Acetyl-3-phenyl-1,4-dihydro-as-triazino[5,6-c]chinolinhydrochlorid mit einem Schmelzpunkt von 256 bis 258° C erhalten.

Diese 6,2 g (0,018 Mol) 4-Acetyl-3-phenyl-1,4-dihydro-as-triazino[5,6-c]chinolinhydrochlorid wurden in 100 $cm^3$ Wasser suspendiert, und der pH-Wert wurde durch Zugabe von konzentrierter Natriumbicarbonatlösung auf 7 eingestellt. Die ausgeschiedenen gelben Kristalle wurden abfiltriert und mit Wasser gewaschen.

So wurden 5,0 g (90,4% der Theorie, bezogen auf das eingesetzte 4-Acetyl-3-phenyl-1,4-dihydro-as-triazino[5,6-c]chinolinhydrochlorid, beziehungsweise 49,1% der Theorie, bezogen auf das eingesetzte 3-Phenyl-1,4-dihydro-as-triazino[5,6-c]chinolinhydrochlorid), 4-Acetyl-3-phenyl-1,4-dihydro-as-triazino[5,6-c]chinolin mit einem Schmelzpunkt von 267 bis 268° C erhalten.

## Beispiel 4

### 1,4-Diacetyl-3-phenyl-1,4-dihydro-as-triazino[5,6-c]chinolin

Es wurden 10,0 g (0,034 Mol) 3-Phenyl-1,4-dihydro-as-triazino[5,6-c]chinolinhydrochlorid 3 Stunden lang mit 50 $cm^3$ Essigsäureanhydrid zum Sieden erhitzt. Die erhaltene Lösung wurde in Wasser eingegossen und die ausgeschiedenen Kristalle wurden abfiltriert und mit Wasser gewaschen.

So wurden 7,0 g (60,3% der Theorie, bezogen auf das eingesetzte 3-Phenyl-1,4-dihydro-as-triazino[5,6-c]chinolinhydrochlorid), 1,4-Diacetyl-3-phenyl-dihydro-as-triazino[5,6-c]chinolin mit einem Schmelzpunkt von 200 bis 202° C erhalten.

Dieses Produkt konnte auch in der Weise hergestellt werden, daß das wie im Beispiel 3 beschrieben erhaltene 4-Acetyl-3-phenyl-1,4-dihydro-as-triazino[5,6-c]chinolin oder dessen Säureadditionssalz mit Essigsäureanhydrid weiter erhitzt wurden.

Beispiel 5

1-Acetyl-3-n-butyl-1,2-dihydro-as-triazino[5,6-c]chinolin

Es wurde 3-n-Butyl-1,2-dihydro-as-triazino[5,6-c]chinolinhydrochlorid mit Essigsäureanhydrid in der im Beispiel 1 beschriebenen Weise acyliert. So wurde zunächst 1-Acetyl-3-n-butyl-1,2-dihydro-as-triazino[5,6-c]chinolinhydrochlorid mit einem Schmelzpunkt von 218 bis 220° C und dann 1-Acetyl-3-n-butyl-1,2-dihydro-as-triazino[5,6-c]chinolin mit einem Schmelzpunkt von 168 bis 170° C in einer Ausbeute von 61% der Theorie, bezogen auf das eingesetzte 3-n-Butyl-1,2-dihydro-as-triazino[5,6-c]chinolinhydrochlorid, erhalten.

Beispiel 6

1-Acetyl-3-n-octyl-1,2-dihydro-as-triazino[5,6-c]chinolin

Es wurde 3-n-Octyl-1,2-dihydro-as-triazino[5,6-c]chinolinhydrochlorid mit Essigsäureanhydrid in der im Beispiel 1 beschriebenen Weise acyliert. So wurde zunächst 1-Acetyl-3-n-octyl-1,2-dihydro-as-triazino[5,6-c]chinolinhydrochlorid mit einem Schmelzpunkt von 210 bis 212° C und dann 1 Acetyl-3-n-octyl-1,2-dihydro-as-triazino[5,6-c]chinolin mit einem Schmelzpunkt von 97 bis 99° C in einer Ausbeute von 63% der Theorie, bezogen auf das eingesetzte 3-n-Octyl-1,2-dihydro-as-triazino[5,6-c]chinolinhydrochlorid, erhalten.

Beispiel 7

1-Acetyl-3-isobutyl-1,2-dihydro-as-triazino[5,6-c]chinolin

Es wurde 3-Isobutyl-1,2-dihydro-as-triazino[5,6-c]chinolinhydrochlorid mit Essigsäureanhydrid in der im Beispiel 1 beschriebenen Weise acyliert. So wurde zunächst 1-Acetyl-3-isobutyl-1,2-dihydro-as-triazino[5,6-c]chinolinhydrochlorid mit einem Schmelzpunkt von 206 bis 208° C und dann 1-Acetyl-3-isobutyl-1,2-dihydro-as-triazino[5,6-c]chinolin mit einem Schmelzpunkt von 234 bis 236° C in einer Ausbeute von 65% der Theorie, bezogen auf das eingesetzte 3-Isobutyl-1,2-dihydro-as-triazino[5,6-c]chinolinhydrochlorid, erhalten.

Beispiel 8

1-Acetyl-3-tert.-butyl-1,2-dihydro-as-triazino[5,6-c]-chinolin

Es wurde 3-tert.-Butyl-1,2-dihydro-as-triazino[5,6-c]chinolinhydrochlorid mit Essigsäureanhydrid in der im Beispiel 1 beschriebenen Weise acyliert. So wurde zunächst 1-Acetyl-3-tert.-butyl-1,2-dihydro-as-triazino[5,6-c]chinolinhydrochlorid mit einem Schmelzpunkt von 268 bis 270° C und dann 1-Acetyl-3-tert.-butyl-1,2-dihydro-as-triazino[5,6-c]chinolin mit einem Schmelzpunkt von 272 bis 274° C in einer Ausbeute von 71% der Theorie, bezogen auf das eingesetzte 3-tert.-Butyl-1,2-dihydro-as-triazino[5,6-c]chinolinhydrochlorid, erhalten.

Beispiel 9

1-Acetyl-3-(3′-methylbut-1′-yl)-1,2-dihydro-as-triazino[5,6-c]chinolin

Es wurde 3-(3′-Methylbut-1′-yl)-1,2-dihydro-as-triazino[5,6-c]chinolinhydrochlorid mit Essigsäureanhydrid in der in der im Beispiel 1 beschriebenen Weise acyliert. So wurde zunächst 1-Acetyl-3-(3′-methylbut-1′-yl)-1,2-di-hydro-as-triazino[5,6-c]chinolinhydrochlorid mit einem Schmelzpunkt von 194 bis 196° C und dann 1-Acetyl-3-(3′-methylbut-1′-yl)-1,2-dihydro-as-triazino[5,6-c]chinolin mit einem Schmelzpunkt von 124 bis 126° C in einer Ausbeute von 52% der Theorie, bezogen auf das eingesetzte 3-(3′-Methylbut-1′-yl)-1,2-dihydro-as-triazino[5, 6-c]chinolinhydrochlorid, erhalten.

Beispiel 10

1-Acetyl-3-(1′-äthylprop-1′-yl)-1,2-di-hydro-as-triazino[5,6-c]chinolin

Es wurde 3-(1′-Äthylprop-1′-yl)-1,2-dihydro-as-triazino[5,6-c]chinolinhydrochlorid mit Essigsäureanhydrid in der im Beispiel 1 beschriebenen Weise acyliert. So wurde zunächst 1-Acetyl-3-(1′-äthylprop-1′-yl)-1,2-dihydro-as-triazino[5,6-c]chinolinhydrochlorid mit einem Schmelzpunkt von 212 bis 214°C und dann 1-Acetyl-3-(1′-äthylprop-1′-yl)-1,2-dihydro-as-triazino[5,6-c]chinolin mit einem Schmelzpunkt von 245 bis 247°C in einer Ausbeute von 75% der Theorie, bezogen auf das eingesetzte 3-(1′-Äthylprop-1′-yl)-1,2-dihydro-as-triazino[5,6-c]chinolinhydrochlorid, erhalten.

Beispiel 11

1-Acetyl-3-(2′,2′-dimethylprop-1′-yl)-1,2-dihydro-as-triazino[5,6-c]chinolin

Es wurde 3-(2′, 2′-Dimethylprop-1′-yl)-1,2-dihydro-as-triazino[5,6-c]chinolinhydrochlorid mit Essigsäureanhydrid in der im Beispiel 1 beschriebenen Weise acyliert. So wurde zunächst 1-Acetyl-3-(2′,2′-dimethylpropyl)-1,2-dihydro-as-triazino[5,6-c]chinolinhydrochlorid mit einem Schmelzpunkt von 235 bis 237°C und dann 1-Acetyl-3-(2′,2′-dimethylprop-1′-yl)-1,2-dihydro-as-triazino[5,6-c]chinolin mit einem Schmelzpunkt von 248 bis 250°C in einer Ausbeute von 45% der Theorie, bezogen auf das eingesetzte 3-(2′,2′-Dimethylprop-1′-yl)-1,2-dihydro-as-triazino[5,6-c]chinolinhydrochlorid, erhalten.

Beispiel 12

1,2-Diacetyl-3-n-butyl-1,2-dihydro-as-triazino[5,6-c]chinolin

Es wurde wie im Beispiel 6 beschrieben erhaltenes 1-Acetyl-3-n-butyl-1,2-dihydro-as-triazino[5,6-c]chinolin mit Essigsäureanhydrid in der im Beispiel 2 beschriebenen Weise acyliert. So wurde 1,2-Diacetyl-3-n-butyl-1,2-dihydro-as-triazino[5,6-c]chinolin mit einem Schmelzpunkt von 104 bis 106°C in einer Ausbeute von 95% der Theorie, bezogen auf das eingesetzte 1-Acetyl-3-n-butyl-1,2-dihydro-as-triazino[5,6-c]chinolin, erhalten.

Beispiel 13

1,2-Diacetyl-3-n-octyl-1,2-dihydro-as-triazino[5,6-c]chinolin

Es wurde wie im Beispiel 7 beschrieben erhaltenes 1-Acetyl-3-n-octyl-1,2-dihydrod-as-triazino[5,6-c]chinolin mit Essigsäureanhydrid in der im Beispiel 2 beschriebenen Weise acyliert. So wurde 1,2-Diacetyl-3-n-octyl-1,2-dihydro-as-triazino[5,6-c]chinolin mit einem Schmelzpunkt von 95 bis 97°C in einer Ausbeute von 80% der Theorie, bezogen auf das eingesetzte 1-Acetyl-3-n-octyl-1,2-dihydro-as-triazino[5,6-c]chinolin, erhalten.

Beispiel 14

1,2-Diacetyl-3-(3′-methylbut-1′-yl)-1,2-dihydro-as-triazino[5,6-c]chinolin

Es wurde wie im Beispiel 10 beschrieben erhaltenes 1-Acetyl-3-(3′-methylbut-1′-yl)-1,2-dihydro-as-triazino[5,6-c]chinolin mit Essigsäureanhydrid in der im Beispiel 2 beschriebenen Weise acyliert. So wurde 1,2-Diacetyl-3-(3′-methylbut-1′-yl)-1,2-dihydro-as-triazino[5,6-c]chinolin mit einem Schmelzpunkt von 116 bis 118°C in einer Ausbeute von 60% der Theorie, bezogen auf das eingesetzte 1-Acetyl-3-(3′-methylbut-1′-yl)-1,2-dihydro-as-triazino[5,6-c]chinolin, erhalten.

Beispiel 15

1,2-Diacetyl-3-isobutyl-1,2-dihydro-as-triazino[5,6-c]chinolin

Es wurde wie im Beispiel 8 beschrieben erhaltenes 1-Acetyl-3-isobutyl-1,2-dihydro-as-triazino[5,6-c]chinolin mit Essigsäureanhydrid in der im Beispiel 2 beschriebenen Weise acyliert. So wurde 1,2-Diacetyl-3-isobutyl-1,2-dihydro-as-triazino[5,6-c]chinolin mit einem Schmelzpunkt von 184 bis 186°C in einer Ausbeute von 58% der Theorie, bezogen auf das eingesetzte 1-Acetyl-3-isobutyl-1,2-dihydro-as-triazino[5,6-c]chinolin, erhalten.

Beispiel 16

1,2-Diacetyl-3-(1'-äthylprop-1'-yl)-1,2-dihydro-as-triazino[5,6-c]chinolin

Es wurde wie im Beispiel 11 beschrieben erhaltenes 1-Acetyl-3-(1'-äthylprop-1'-yl)-1,2-dihydro-as-triazino[5,6-c]chinolin mit Essigsäureanhydrid in der im Beispiel 2 beschriebenen Weise acyliert. So wurde 1,2-Diacetyl-3-(1'-äthylprop-1'-yl)-1,2-dihydro-as-triazino[5,6-c]chinolin mit einem Schmelzpunkt von 124 bis 126° C in einer Ausbeute von 67% der Theorie, bezogen auf das eingesetzte 1-Acetyl-3-(1'-äthylprop-1'-yl)-1,2-dihydro-as-triazino[5,6-c]chinolin, erhalten.

**Patentansprüche für die Vertragsstaaten: BE, CH/Li, DE, FR, GB, IT, NL, SE**

1. Dihydro-as-triazino[5,6-c]chinolinderivate der allgemeinen Formel

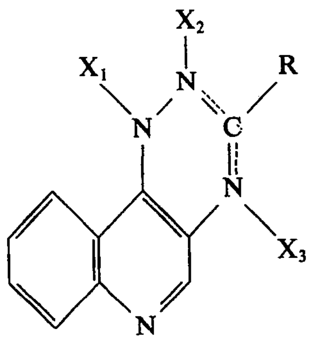

(I)

worin

R für einen Alkylrest mit 4, 5 oder 8 Kohlenstoffatomen oder einen Phenylrest steht und
$X_1$ ein Wasserstoffatom oder einen Acetylrest bedeutet und

entweder $X_2$ oder $X_3$ Wasserstoff oder einen Acetylrest darstellt und
das andere von $X_2$ und $X_3$ keine Bedeutung hat und
die gestrichelten Linien jeweils 1 chemische Bindung bedeuten, wobei nur entweder die eine oder die andere vorliegt,
mit der weiteren Maßgabe, daß falls $X_1$ für Wasserstoff steht, keines von $X_2$ und $X_3$ Wasserstoff bedeutet,

sowie ihre Säureadditionssalze.

2. 1-Acetyl-3-n-pentyl-1,2-dihydro-as-triazino[5,6-c]chinolin.
3. 4-Acetyl-3-phenyl-1,4-dihydro-as-triazino[5,6-c]chinolin.
4. 1-Acetyl-3-n-butyl-1,2-dihydro-as-triazino[5,6-c]chinolin.
5. 1-Acetyl-3-n-octyl-1,2-dihydro-as-triazino[5,6-c]chinolin.
6. 1-Acetyl-3-isobutyl-1,2-dihydro-as-triazino[5,6-c]chinolin.
7. 1-Acetyl-3-(3'-methylbut-1'-yl)-1,2-dihydro-as-triazino[5,6-c]chinolin.
8. 1-Acetyl-3-tert.-butyl-1,2-dihydro-as-triazino[5,6-c]chinolin.
9. Verfahren zur Herstellung der Verbindungen nach Anspruch 1 bis 8, dadurch gekennzeichnet, daß man in an sich bekannter Weise Säureadditionssalze von Dihydro-as-triazino[5,6-c]chinolinderivaten der allgemeinen Formel

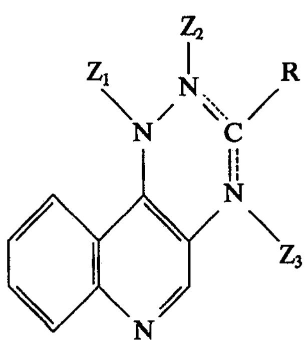

(II)

worin

R wie im Anspruch 1 festgelegt ist und
$Z_1$ für Wasserstoff steht und

entweder $Z_2$ oder $Z_3$ Wasserstoff bedeutet und
das andere von $Z_2$ und $Z_3$ keine Bedeutung hat und
die gestrichelten Linien jeweils 1 chemische Bindung bedeuten, wobei nur entweder die eine oder die andere vorliegt,

mit Essigsäure und/oder acetylierungsaktiven Derivaten derselben als Acetylierungsmitteln acetyliert und gegebenenfalls möglicherweise erhaltene Monoacetyldihydro-as-triazino[5, 6-c]chinolinderivate der allgemeinen Formel I beziehungsweise Säureadditionssalze derselben, gegebenenfalls nach Freisetzen der ersteren aus den letzteren beziehungsweise Überführen der letzteren in die ersteren, durch weiteres Acetylieren mit Essigsäure und/oder acetylierungsaktiven Derivaten derselben als Acetylierungsmitteln in Diacetyldihydro-as-triazino[5,6-c]chinolinderivate der allgemeinen Formel I beziehungsweise Säureadditionssalze derselben überführt, worauf man in an sich bekannter Weise gegebenenfalls die erhaltenen Dihydro-as-triazino[5,6-c]chinolinderivate der allgemeinen Formel I mit anorganischen oder organischen Säuren in Säureadditionssalze überführt beziehungsweise gegebenenfalls die erhaltenen Säureadditionssalze der Dihydro-as-triazino[5, 6-c]chinolinderivate der allgemeinen Formel I mit Basen in die freien Dihydro-as-triazino[5,6-c]chinolinderivate der allgemeinen Formel I oder in andere Säureadditionssalze überführt.

10. Verfahren nach Anspruch 9, dadurch gekennzeichnet, daß man als acetylierungsaktive Derivate der Essigsäure Carbonsäureanhydride der allgemeinen Formel

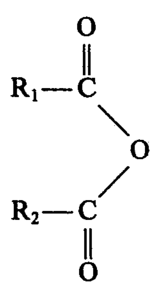

(III)

worin $R_1$ und $R_2$ unabhängig voneinander Wasserstoff beziehungsweise Methylreste bedeuten, einsetzt.

11. Arzneimittel, gekennzeichnet durch einen Gehalt an 1 oder mehrerer Verbindung(en) nach Anspruch 1 bis 8 als Wirkstoff(e), gegebenenfalls zusammen mit 1 oder mehreren inerten festen oder flüssigen Trägerstoff(en).

**Patentansprüche für den Vertragsstaat: AT**

1. Verfahren zur Herstellung von Dihydro-as-triazino[5,6-c]chinolinderivaten der allgemeinen Formel

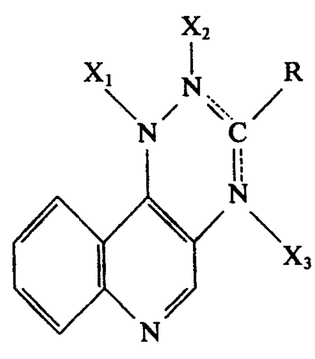

(I)

worin

R für einen Alkylrest mit 4, 5 oder 8 Kohlenstoffatomen oder einen Phenylrest steht und
$X_1$ ein Wasserstoffatom oder einen Acetylrest bedeutet und

entweder $X_2$ oder $X_3$ Wasserstoff oder einen Acetylrest darstellt und
das andere von $X_2$ und $X_3$ keine Bedeutung hat und

die gestrichelten Linien jeweils 1 chemische Bindung bedeuten, wobei nur entweder die eine oder die andere vorliegt,
mit der weiteren Maßgabe, daß falls $X_1$ für Wasserstoff steht, keines von $X_2$ und $X_3$ Wasserstoff bedeutet,

sowie ihren Säureadditionssalzen, dadurch gekennzeichnet, daß man in an sich bekannter Weise Säureadditionssalze von Dihydro-as-triazino[5,6-c]chinolinderivaten der allgemeinen Formel

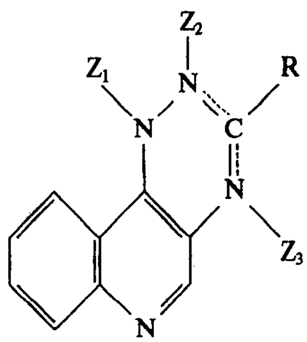

(II)

worin

R wie oben festgelegt ist und
$Z_1$ für Wasserstoff steht und

entweder $Z_2$ oder $Z_3$ Wasserstoff bedeutet und
das andere von $Z_2$ und $Z_3$ keine Bedeutung hat und
die gestrichelten Linien jeweils 1 chemische Bindung bedeuten, wobei nur entweder die eine oder die andere vorliegt,

mit Essigsäure und/oder acetylierungsaktiven Derivaten derselben als Acetylierungsmitteln acetyliert und gegebenenfalls möglicherweise erhaltene Monoacetyldihydro-as-triazino[5,6-c]chinolinderivate der allgemeinen Formel I beziehungsweise Säureadditionssalze derselben, gegebenenfalls nach Freisetzen der ersteren aus den letzteren beziehungsweise Überführen der letzteren in die ersteren, durch weiteres Acetylieren mit Essigsäure und/oder acetylierungsaktiven Derivaten derselben als Acetylierungsmitteln in Diacetyldihydro-as-triazino[5,6-c]chinolinderivate der allgemeinen Formel I beziehungsweise Säureadditionssalze derselben überführt, worauf man in an sich bekannter Weise gegebenenfalls die erhaltenen Dihydro-as-triazino[5,6-c]chinolinderivate der allgemeinen Formel I mit anorganischen oder organischen Säuren in Säureadditionssalze überführt beziehungsweise gegebenenfalls die erhaltenen Säureadditionssalze der Dihydro-as-triazino[5,6-c]chinolinderivate der allgemeinen Formel I mit Basen in die freien Dihydro-as-triazino[5,6-c]chinolinderivate der allgemeinen Formel I oder in andere Säureadditionssalze überführt.

2. Verfahren nach Anspruch 1, dadurch gekennzeichnet, daß man als acetylierungsaktive Derivate der Essigsäure Carbonsäureanhydride der allgemeinen Formel

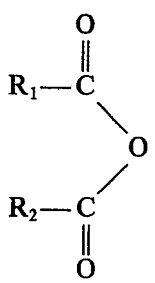

(III)

worin $R_1$ und $R_2$ unabhängig voneinander Wasserstoff beziehungsweise Methylreste bedeuten, einsetzt.

3. Verfahren nach Anspruch 1 oder 2, dadurch gekennzeichnet, daß man 1-Acetyl-3-n-pentyl-1,2-dihydro-as-triazino[5,6-c]chinolin herstellt.

4. Verfahren nach Anspruch 1 oder 2, dadurch gekennzeichnet, daß man 4-Acetyl-3-phenyl-1,4-dihydro-as-triazino[5,6-c]chinolin herstellt.

5. Verfahren nach Anspruch 1 oder 2, dadurch gekennzeichnet, daß man 1-Acetyl-3-n-butyl-1,2-dihydro-as-triazino[5, 6-c]chinolin herstellt.

6. Verfahren nach Anspruch 1 oder 2, dadurch gekennzeichnet, daß man 1-Acetyl-3-n-octyl-1,2-dihydro-as-triazino[5,6-c]chinolin herstellt.

7. Verfahren nach Anspruch 1 oder 2, dadurch gekennzeichnet, daß man 1-Acetyl-3-isobutyl-1,2-dihydro-as-triazino[5,6-c]chinolin herstellt.

8. Verfahren nach Anspruch 1 oder 2, dadurch gekennzeichnet, daß man 1-Acetyl-3-(3′-methylbut-1′-yl)-1,2-dihydro-as-triazino[5,6-c]chinolin herstellt.

9. Verfahren nach Anspruch 1 oder 2, dadurch gekennzeichnet, daß man 1-Acetyl-3-tert.-butyl-1,2-dihydro-as-triazino[5,6-c]chinolin herstellt.

**Claims for the Contracting states: BE, CH/LI, DE, FR, GB, IT, NL, SE**

1. Dihydro-as-triazino[5,6-c]quinoline derivatives having the general formula

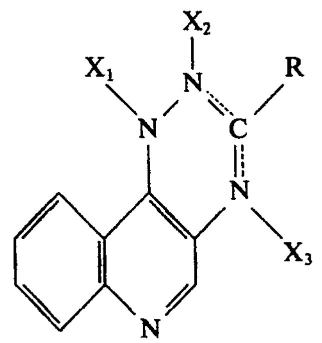

(I)

wherein

R represents an alkyl radical having 4, 5 or 8 carbon atoms or a phenyl radical and
$X_1$ means a hydrogen atom or an acetyl radical and

either $X_2$ or $X_3$ represents hydrogen or an acetyl radical and
the other of $X_2$ and $X_3$ has no meaning and
the dotted lines mean 1 chemical bond each only either the one or the other being present
with the further definition that in case $X_1$ represents hydrogen none of $X_2$ and $X_3$ means hydrogen

as well as their acid addition salts.

2. 1-Acetyl-3-n-pentyl-1,2-dihydro-as-triazino[5,6-c]quinoline.

3. 4-Acetyl-3-phenyl-1,4-dihydro-as-triazino[5,6-c]quinoline.

4. 1-Acetyl-3-n-butyl-1,2-dihydro-as-triazino[5,6-c]quinoline.

5. 1-Acetyl-3-n-octyl-1,2-dihydro-as-triazino[5,6-c]quinoline.

6. 1-Acetyl-3-isobutyl-1,2-dihydro-as-triazino[5,6-c]quinoline.

7. 1-Acetyl-3-(3′-methylbut-1′-yl)-1,2-dihydro-as-triazino[5,6-c]quinoline.

8. 1-Acetyl-3-tert.-butyl-1,2-dihydro-as-triazino[5,6-c]quinoline.

9. A process for preparing the compounds according to claims 1 to 8, characterized in that in a manner known per se one acetylizes acid addition salts of dihydro-as-triazino[5,6-c]quinoline derivatives having the general formula

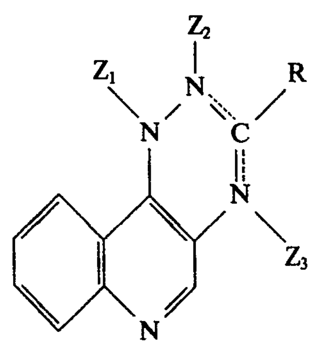

(II)

wherein

R is as defined in claim 1 and
$Z_1$ represents hydrogen and

either $Z_2$ or $Z_3$ means hydrogen and
the other of $Z_2$ and $Z_3$ has no meaning and

the dotted lines mean 1 chemical bond each only either the one or the other being present

with acetic acid and/or derivatives of it active regarding acetylation as acetylating agents and optionally one converts possibly obtained monoacetyl-dihydro-as-triazino[5,6-c]quinoline derivatives having the general formula I or acid addition salts thereof, respectively, optionally after liberating the former from the latter or converting the latter into the former, respectively, by further acetylation with acetic acid and/or derivatives of it active regarding acetylation as acetylating agents into diacetyldihydro-as-triazino[5,6-c]quinoline derivatives having the general formula I or acid addition salts thereof, respectively, whereafter in a manner known per se optionally one converts the obtained dihydro-as-triazino[5,6-c]quinoline derivatives having the general formula I with inorganic or organic acids into acid addition salts or optionally one converts the obtained acid addition salts of the dihydro-as-triazino[5,6-c]quinoline derivatives having the general formula I with bases into the free dihydro-as-triazino[5,6-c]quinoline derivatives having the general formula I or in other acid addition salts, respectively.

10. A process according to claim 9, characterized in that one uses as derivatives of the acetic acid active regarding acetylation carbonic acid anhydrides having the general formula

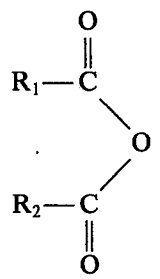

(III)

wherein $R_1$ and $R_2$ independently of each other mean hydrogen or methyl radicals, respectively.

11. Medicaments, characterized by a content of 1 or several compound(s) according to claims 1 to 8 as active principle(s), optionally together with 1 or several inert solid or liquid carrier material(s).

**Claims for the Contracting state: AT**

1. A process for preparing dihydro-as-triazino[5,6-c]quinoline derivatives having the general formula

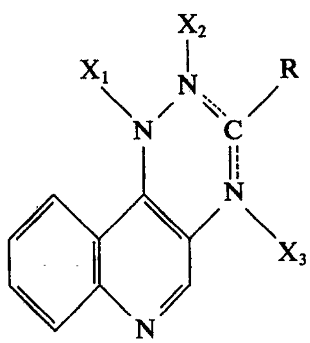

(I)

wherein

R represents an alkyl radical having 4, 5 or 8 carbon atoms or a phenyl radical and
$X_1$ means a hydrogen atom or an acetyl radical and

either $X_2$ or $X_3$ represents hydrogen or an acetyl radical and
the other of $X_2$ and $X_3$ has no meaning and
the dotted lines mean 1 chemical bond each only either the one or the other being present
with the further definition that in case $X_1$ represents hydrogen none of $X_2$ and $X_3$ means hydrogen

as well as their acid addition salts, characterized in that in a manner known per se one acetylizes acid addition salts of dihydro-as-triazino[5,6-c]quinoline derivatives having the general formula

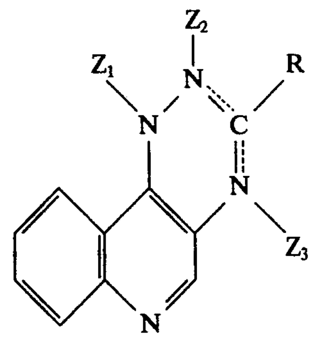

(II)

wherein

R is as above defined and
$Z_1$ represents hydrogen and

either $Z_2$ or $Z_3$ means hydrogen and
the other of $Z_2$ and $Z_3$ has no meaning and
the dotted lines mean 1 chemical bond each only either the one or the other being present

with acetic acid and/or derivatives of it active regarding acetylation as acetylating agents and optionally one converts possibly obtained monoacetyl-dihydro-as-triazino[5,6-c]quinoline derivatives having the general formula I or acid addition salts thereof, respectively, optionally after liberating the former from the latter or converting the latter into the former, respectively, by further acetylation with acetic acid and/or derivatives of it active regarding acetylation as acetylating agents into diacetyldihydro-as-triazino[5,6-c]quinoline derivatives having the general formula I or acid addition salts thereof, respectively, whereafter in a manner known per se optionally one converts the obtained dihydro-as-triazino-[5,6-c]quinoline derivatives having the general formula I with inorganic or organic acids into acid addition salts or optionally one converts the obtained acid addition salts of the dihydro-as-triazino[5,6-c]quinoline derivatives having the general formula I with bases into the free dihydro-as-triazino[5,6-c]quinoline derivatives having the general formula I or in other acid addition salts, respectively.

2. A process according to claim 1, characterized in that one uses as derivatives of the acetic acid active regarding acetylation carbonic acid anhydrides having the general formula

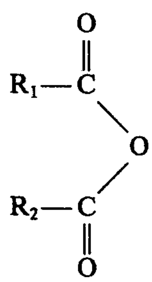

(III)

wherein $R_1$ and $R_2$ independently of each other mean hydrogen or methyl radicals, respectively.

3. A process according to claim 1 or 2, characterized in that one prepares 1-acetyl-3-n-pentyl-1,2-dihydro-as-triazino[5,6-c]quinoline.

4. A process according to claim 1 or 2, characterized in that one prepares 4-acetyl-3-phenyl-1,4-dihydro-as-triazino[5,6-c]quinoline.

5. A process according to claim 1 or 2, characterized in that one prepares 1-acetyl-3-n-butyl-1,2-dihydro-as-triazino[5,6-c]quinoline.

6. A process according to claim 1 or 2, characterized in that one prepares 1-acetyl-3-n-octyl-1,2-dihydro-as-triazino[5,6-c]quinoline.

7. A process according to claim 1 or 2, characterized in that one prepares 1-acetyl-3-isobutyl-1,2-dihydro-as-triazino[5,6-c]quinoline.

8. A process according to claim 1 or 2, characterized in that one prepares 1-acetyl-3-(3'-methylbut-1'-yl)-1,2-dihydro-as-triazino[5,6-c]quinoline.

9. A process according to claim 1 or 2, characterized in that one prepares 1-acetyl-3-tert.-butyl-1,2-dihydro-as-triazino[5,6-c]quinoline.

**Revendications pour les Etats contractants: BE, CH/LI, DE, FR, GB, IT, NL, SE**

1. Dérivés de dihydro-as-triazino(5,6-c)quinoléine, de formule générale:

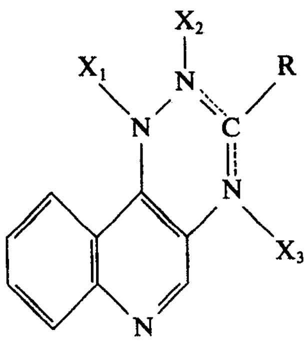

(I)

dans laquelle:

R représente un radical alkyle de 4, 5 ou 8 atomes de carbone ou un radical phényle, et
$X_1$ représente un atome d'hydrogène ou un radical acétyle, et

soit $X_2$ soit $X_3$ représente l'hydrogène ou un radical acétyle, et
l'autre de $X_2$ et $X_3$ n'a pas de signification, et
les tiretés représentent chacun 1 liaison chimique, l'une seulement ou l'autre seulement étant présente,
étant entendu en outre que si $X_1$ représente l'hydrogène, aucun de $X_2$ et $X_3$ ne représente l'hydrogène,

ainsi que leurs sels d'addition d'acide.

2. 1-Acétyl-3-n-pentyl-1,2-dihydro-as-triazino(5,6-c)quinoléine.
3. 4-Acétyl-3-phényl-1,4-dihydro-as-triazino(5,6-c)quinoléine.
4. 1-Acétyl-3-n-butyl-1,2-dihydro-as-triazino(5,6-c)quinoléine.
5. 1-Acétyl-3-n-octyl-1,2-dihydro-as-triazino(5,6-c)quinoléine.
6. 1-Acétyl-3-isobutyl-1,2-dihydro-as-triazino(5,6-c)quinoléine.
7. 1-Acétyl-3-(3'-méthylbut-1'-yl)-1,2-dihydro-as-triazino(5,6-c) quinoléine.
8. 1-Acétyl-3-tertiobutyl-1,2-dihydro-as-triazino(5,6-c)quinoléine.
9. Procédé de préparation des composés selon les revendications 1 à 8, caractérisé par le fait que de manière en elle-même connue, on acétyle des sels d'addition d'acide de dérivés de dihydro-as-triazino(5,6-c)quinoléine de formule générale:

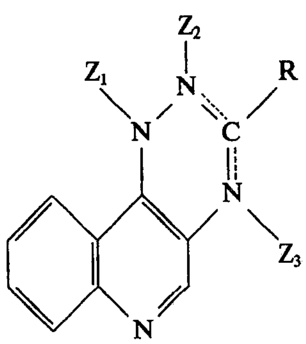

(II)

dans laquelle,

R est tel que défini à la revendication 1, et
$Z_1$ représente l'hydrogène, et

soit $Z_2$ soit $Z_3$ représente l'hydrogène, et
l'autre de $Z_2$ et $Z_3$ n'a pas de signification et
les tiretés représentent chacun 1 liaison chimique, l'une seulement ou l'autre seulement étant présente,

au moyen d'acide acétique et/ou de dérivés de celui-ci qui sont actifs pour l'acétylation comme agents acétylants et qu'éventuellement on convertit les dérivés de monoacétyldihydro-as-triazino(5,6-c)quinoléine de la formule générale I ou sels d'addition d'acide de ceux-ci, éventuellement obtenus, éventuellement après avoir libéré les premiers en partant des seconds ou après avoir converti les

seconds en les premiers, en dérivés de diacétyl-dihydro-as-triazino(5,6-c)quinoléine de la formule générale I ou en sels d'addition d'acide de ceux-ci, par une nouvelle acétylation au moyen d'acide acétique et/ou de dérivés de celui-ci qui sont actifs pour l'acétylation comme agents acétylants, après quoi, de manière en elle-même connue, on convertit éventuellement les dérivés de dihydro-as-triazino(5,6-c)quinoléine obtenus, de la formule générale I, en sels d'addition d'acide au moyen d'acides minéraux ou organiques, ou, éventuellement, on convertit les sels d'addition d'acide des dérivés de dihydro-as-triazino(5,6-c)quinoléine de la formule générale I que l'on a obtenus, au moyen de bases, en les dérivés libres de dihydro-as-triazino(5,6-c)quinoléine de la formule générale I ou en d'autres sels d'addition d'acide.

10. Procédé selon la revendication 9, caractérisé par le fait que comme dérivés de l'acide acétique actifs pour l'acylation, on utilise des anhydrides d'acide carboxylique de formule générale:

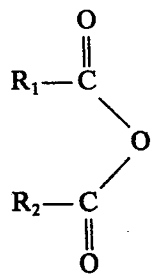

(III)

dans laquelle $R_1$ et $R_2$ représentent, indépendamment l'un de l'autre, l'hydrogène ou des radicaux méthyle.

11. Médicaments caractérisés par une teneur en un ou plusieurs composés selon les revendications 1 à 8, comme substance active, éventuellement en même temps qu'un ou plusieurs véhicules inertes solides ou liquides.

**Revendications pour l'Etat contractant: AT**

1. Procédé de préparation des dérivés de dihydro-as-triazino(5, 6-c)quinoléine, de formule générale:

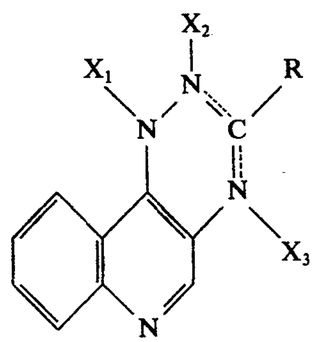

(I)

dans laquelle:

R représente un radical alkyle de 4, 5 ou 8 atomes de carbone ou un radical phényle, et
$X_1$ représente un atome d'hydrogène ou un radical acétyle, et

soit $X_2$ soit $X_3$ représente l'hydrogène ou un radical acétyle, et
l'autre de $X_2$ et $X_3$ n'a pas de signification, et
les tiretés représentent chacun 1 liaison chimique, l'une seulement ou l'autre seulement étant présente,
étant entendu en outre que si $X_1$ représente l'hydrogène, aucun de $X_2$ et $X_3$ ne représente l'hydrogène,

ainsi que leurs sels d'addition d'acide, caractérisé par le fait que de manière en elle-même connue, on acétyle des sels d'addition d'acide de dérivés de dihydro-as-triazino(5,6-c)quinoléine de formule générale:

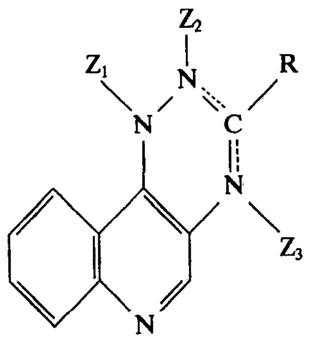

(II)

dans laquelle,

R est tel que défini ci-dessus, et
$Z_1$ représente l'hydrogène, et

soit $Z_2$ soit $Z_3$ représente l'hydrogène, et
l'autre de $Z_2$ et $Z_3$ n'a pas de signification et les tiretés représentent chacun 1 liaison chimique, l'une seulement ou l'autre seulement étant présente, au moyen d'acide acétique et/ou de dérivés de celui-ci qui sont actifs pour l'acétylation comme agents acétylants et qu'éventuellement on convertit les dérivés de monoacétyldihydro-as-triazino(5,6-c) quinoléine de la formule générale I ou sels d'addition d'acide de ceux-ci, éventuellement obtenus, éventuellement après avoir libéré les premiers en partant des seconds ou après avoir converti les seconds en les premiers, en dérivés de diacétyl-dihydro-as-triazino(5,6-c)quinoléine de la formule générale I ou en sels d'addition d'acide de ceux-ci, par une nouvelle acétylation au moyen d'acide acétique et/ou de dérivés de celui-ci qui sont actifs pour l'acétylation comme agents acétylants, après quoi, de manière en elle-même connue, on convertit éventuellement les dérivés de dihydro-as-triazino(5,6-c)quinoléine obtenus, de la formule générale I, en sels d'addition d'acide au moyen d'acides minéraux ou organiques, ou, éventuellement, on convertit les sels d'addition d'acide des dérivés de dihydro-as-triazino(5,6-c)quinoléine de la formule générale I que l'on a obtenus, au moyen de bases, en les dérivés libres de dihydro-as-triazino(5,6-c)quinoléine de la formule générale I ou en d'autres sels d'addition d'acide.

2. Procédé selon la revendication 1, caractérisé par le fait que comme dérivés de l'acide acétique actifs pour l'acylation, on utilise des anhydrides d'acide carboxylique de formule générale:

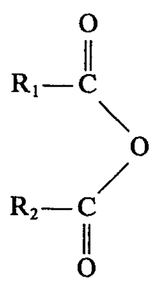

(III)

dans laquelle $R_1$ et $R_2$ représentent, indépendamment l'un de l'autre, l'hydrogène ou des radicaux méthyle.

3. Procédé selon la revendication 1 ou 2, caractérisé par le fait qu'on prépare 1-acétyl-3-n-pentyl-1,2-dihydro-as-triazino(5,6-c)quinoléine.

4. Procédé selon la revendication 1 ou 2, caractérisé par le fait qu'on prépare 4-acétyl-3-phényl-1,4-dihydro-as-triazino(5,6-c)quinoléine.

5. Procédé selon la revendication 1 ou 2, caractérisé par le fait qu'on prépare 1-acétyl-3-n-butyl-1,2-dihydro-as-triazino(5,6-c)quinoléine.

6. Procédé selon la revendication 1 ou 2, caractérisé par le fait qu'on prépare 1-acétyl-3-n-octyl-1,2-dihydro-as-triazino(5,6-c)quinoléine.

7. Procédé selon la revendication 1 ou 2, caractérisé par le fait qu'on prépare 1-acétyl-3-isobutyl-1,2-dihydro-as-triazino(5,6-c)quinoléine.

8. Procédé selon la revendication 1 ou 2, caractérisé par le fait qu'on prépare 1-acétyl-3-(3'-méthyl-but-1'-yl)-1,2-dihydro-as-triazino(5,6-c)quinoléine.

9. Procédé selon la revendication 1 ou 2, caractérisé par le fait qu'on prépare 1-acétyl-3-tertiobutyl-1,2-dihydro-as-triazino(5,6-c)quinoléine.